# EUROPÄISCHE PATENTSCHRIFT

(11) **EP 4 330 663 B1**
(45) Veröffentlichungstag und Bekanntmachung des Hinweises auf die Patenterteilung: **25.03.2026**
(21) Anmeldenummer: 22725881.1
(22) Anmeldetag: 27.04.2022
(51) Int. Cl.: G01N 30/44, B01D 15/18

(54) **VERFAHREN UND VORRICHTUNG ZUM TRENNEN EINER STOFFMISCHUNG UND ENTSPRECHENDES COMPUTERPROGRAMMPRODUKT**
METHOD AND APPARATUS FOR SEPARATING A MIXTURE AND CORRESPONDING COMPUTER PROGRAM PRODUCT
PROCÉDÉ ET DISPOSITIF DE SÉPARATION D'UN MÉLANGE ET PRODUIT DE PROGRAMME INFORMATIQUE

(30) Priorität: 27.04.2021 LU 500093; 24.08.2021 LU 500567
(43) Veröffentlichungstag der Anmeldung: 06.03.2024
(73) Patentinhaber: K.D. Pharma Bexbach GmbH, 66450 Bexbach (DE)
(72) Erfinder: GIESSELMANN, Gideon, 54456 Tawern (DE); FRONZONI, Roberto, 66424 Homburg (DE); RAMOS-TERCERO, Elia, 4053 Basel (CH); SHOPOVA-GOSPODINOVA, Denitsa, 66901 Schönenberg-Kübelberg (DE)
(74) Vertreter: Patentanwälte Bernhardt / Wolff Partnerschaft mbB
(86) Internationale Anmeldenummer: PCT/EP2022/061251
(87) Internationale Veröffentlichungsnummer: WO 2022/229284

(56) Entgegenhaltungen:
- EP-A1- 3 455 213
- DE-T2- 69 632 076
- US-A1- 2012 330 043
- SYLVIA FEIL: "Fettsäuren geben Signal für Rückzug von Entzündung", CHEMIE IN UNSERER ZEIT, VERLAG CHEMIE, WEINHEIM, DE, vol. 48, no. 4, 5 August 2014 (2014-08-05), pages 244, XP071178688, ISSN: 0009-2851, DOI: 10.1002/CIUZ.201490045

## Beschreibung

Die Erfindung betrifft ein Verfahren zum Trennen eines ersten Stoffes aus einer Stoffmischung mittels Chromatographie, wobei die Stoffmischung zumindest einen zweiten Stoff aufweist, der bei der Chromatographie einer größeren Retention unterliegt als der erste Stoff, und einen dritten Stoff umfasst, der bei der Chromatographie einer geringeren Retention unterliegt als der der erste Stoff, wobei die Stoffmischung einer Vorrichtung zur Chromatographie zugeführt wird, die mehrere miteinander verbundene Chromatographiesäulen umfasst, der Vorrichtung die Stoffmischung und ein Eluent zugeführt werden sowie der zweite Stoff als Extrakt und der erste Stoff als Raffinat entnommen werden.

WO 2017/194173 A1 betrifft ein Verfahren zur Reinigung von Cannabinoidverbindungen unter Verwendung von simulierter Fließbettchromatographie, wobei die Cannabinoidverbindungen in dem Extrakt und/oder dem Raffinat mit einer Gesamtmenge an isomeren Verunreinigungen unterhalb der Nachweisgrenze erhalten werden.

US 2012/0330043 A1 beschreibt ein chromatographisches Trennverfahren zur Gewinnung eines mehrfach ungesättigten Fettsäure (PUFA)-Produkts aus einer Beschickungsmischung, wobei das Verfahren das Einführen der Beschickungsmischung in eine simulierte oder tatsächliche Fließbett-Chromatographievorrichtung mit einer Vielzahl von verbundenen Chromatographiesäulen, die als Elutionsmittel einen wässrigen Alkohol enthalten, umfasst.

Chromatographieverfahren der obengenannten Art sind durch Benutzung bekannt. Eine zu trennende Stoffmischung wird durch die Chromatographiesäule geleitet, die eine stationäre Phase aufweist, welche z.B. eine sog. Packung aus porösem Material umfasst. Die verschiedenen Stoffe der Stoffmischung unterliegen beim Durchfluss durch die stationäre Phase unterschiedlichen Retentionen, d.h. sie durchströmen die stationäre Phase aufgrund unterschiedlich starker Wechselwirkungen in der stationären Phase unterschiedlich schnell. Dies macht die Trennung möglich. Während es möglich ist, Chromatographie im sog. Batch-Verfahren durchzuführen, bei welchem die Trennung immer in einem einzigen Schritt erfolgt, sind für eine kontinuierliche Durchführung der Chromatographie Verfahren entwickelt worden, bei denen mehrere der Chromatographiesäulen miteinander verbunden sind. Solche Chromatographieverfahren, die im industriellen Maßstab durchgeführt werden, sind die True Moving Bed Chromatographie (TMB) und die Simulated Moving Bed Chromatographie (SMB).

Bei der SMB-Chromatographie sind mehrere der Chromatographiesäulen in Reihe miteinander verbunden. An unterschiedlichen Verbindungsstellen zwischen den Chromatographiesäulen werden das Stoffgemisch und ein Elutionsmittel eingegeben. Ebenfalls an anderen Verbindungsstellen werden ein Raffinat und ein Extrakt entnommen, wobei als Raffinat der Stoff oder ggf. die Stoffe entnommen werden, die bei der Chromatographie einer geringeren Retention unterliegen, und als Extrakt der Stoff bzw. die Stoffe entnommen werden, die bei der Chromatographie einer größeren Retention unterliegen. Folglich lassen sich die Stoffgemische mittels der bekannten Chromatographieverfahren je nach Handhabung der Chromatographie in zwei unterschiedliche Stoffuntergemische trennen, wobei ein erstes Stoffuntergemisch Stoffe enthält, die einer geringeren Retention unterliegen, und ein zweites Stoffuntergemisch Stoffe enthält, die einer größeren Retention unterliegen.

Der Erfindung liegt die Aufgabe zugrunde, die Stofftrennung mittels der Chromatographie flexibler durchführen zu können.

Erfindungsgemäß wird diese Aufgabe dadurch gelöst, dass der dritte Stoff als Extrakt entnommen wird.

Durch die Erfindung wird es möglich, einen Stoff aus der Stoffmischung herauszutrennen, auch wenn in der Stoffmischung andere Stoffe vorhanden sind, die bei der Chromatographie jeweilig einer größeren oder einer kleineren Retention als der herauszutrennende Stoff unterliegen. Während es bisher notwendig war, nacheinander mehrere Chromatographien durchzuführen um zu einem vergleichbaren Ergebnis zukommen, lässt sich der zu trennende Stoff mittels des erfindungsgemäßen Verfahrens mittels Durchführung einer einzigen Chromatographie trennen. Der Aufwand zur Durchführung des Trennverfahrens verringert sich erheblich.

In einer Ausgestaltung der Erfindung bilden die Chromatographiesäulen Zonen, wobei
a) eine erste Zone zwischen einer Einrichtung zur Zuleitung des Eluenten und einer Einrichtung zur Ableitung des Extrakts,
b) eine zweite Zone zwischen einer Einrichtung zur Ableitung des Extrakts und einer Einrichtung zur Zuleitung des Stoffgemischs,
c) eine dritte Zone zwischen einer Einrichtung zur Zuleitung des Stoffgemischs und einer Einrichtung zur Ableitung des Raffinats, und
d) eine vierte Zone zwischen einer Ableitung des Raffinats und einer Einrichtung zur Zuleitung des Eluenten gebildet ist.

Zweckmäßigerweise werden Stoffflüsse, insbesondere die Zu- und/oder Abflüsse, über die genannten Zu- und/oder Ableitungseinrichtungen derart eingestellt, dass in einer Zone zwischen einer Einrichtung zur Ableitung des Raffinates und einer Einrichtung zur Zuleitung des Eluenten ein derart großer Fluss vorliegt, dass der dritte Stoff als Extrakt entnommen werden kann. Bei einer derartigen Einstellung der Chromatographievorrichtung wird der dritte Stoff, anders als es bei den bekannten Chromatographieverfahren üblich ist, in die Zone I gedrückt, aus welcher der Extrakt entnommen wird. Während der dritte Stoff bei Anwendung der bekannten Chromatographieverfahren aufgrund seiner im Vergleich zum ersten Stoff geringeren Retention als Raffinat entnommen würde, wird er erfindungsgemäß als Extrakt entnommen.

Als besonders vorteilhaft hat es sich erwiesen, den dritten Stoff gemeinsam mit dem zweiten Stoff als Extrakt zu entnehmen.

In einer Ausführungsform der Erfindung weisen die Einrichtung zur Zuleitung des Eluenten, die Einrichtung zur Ableitung des Extrakts, die Einrichtung zur Zuleitung des Stoffgemischs und die Einrichtung zur Ableitung des Raffinats mehrere Zuleitungs- bzw. Ableitungsanschlüsse auf, die zwischen verschiedenen der Chromatographiesäulen angeordnet sind. Die Zuleitungs- bzw. Ableitungsanschlüsse sind vorzugsweise mit Ventilen versehen, mittels derer sich die jeweiligen Stoffflüsse, insbesondere die Zu- und/oder Abflüsse, des Stoffgemischs, des Eluenten, des Extrakts und/oder des Raffinats durch die jeweiligen Zuleitungs- bzw. Ableitungsanschlüsse einstellen lassen. Erfindungsgemäß werden die jeweiligen Stoffflüsse eingestellt derart, dass die Zonen in der Vorrichtung wechselnd in unterschiedlichen der Chromatographiesäulen gebildet werden. Auf diese Weise wird es möglich, das erfindungsgemäße Chromatographieverfahren als TMB- oder als SMB-Verfahren durchzuführen.

In einer bevorzugten Ausführungsform der Erfindung ist ein Gehalt an dem ersten Stoff im Raffinat größer als ein Gehalt des ersten Stoffs in der Stoffmischung und vorzugsweise ein Gehalt an dem zweiten und/oder dem dritten Stoff im Extrakt größer als der Gehalt des zweiten bzw. des dritten Stoffs in der Stoffmischung. Zweckmäßigerweise wird der Gehalt an dem ersten Stoff im Raffinat maximiert und insbesondere der Gehalt an dem zweiten und/oder dem dritten Stoff im Extrakt maximiert.

Zweckmäßigerweise ist ein Gehalt an dem ersten Stoff im Extrakt kleiner als der Gehalt des ersten Stoffs in der Stoffmischung und vorzugsweise ein Gehalt an dem zweiten und/oder dem dritten Stoff im Raffinat kleiner als Gehalt des zweiten bzw. dritten Stoffs in der Stoffmischung.

Vorzugsweise ist ein Gehalt an dem ersten Stoff im Extrakt kleiner als ein Gehalt des ersten Stoffs im Raffinat und vorzugsweise ein Gehalt an dem zweiten und/oder dem dritten Stoff im Raffinat größer als Gehalt des zweiten bzw. dritten Stoffs im Extrakt.

In einer besonders bevorzugten Ausgestaltung der Erfindung werden die jeweiligen Stoffflüsse derart eingestellt, dass zumindest eines des obengenannten Gehaltsverhältnisse im Raffinat bzw. Extrakt erreicht wird. Vorzugsweise erfolgt die Einstellung kontinuierlich, besonders bevorzugt als Regelung und/oder Steuerung.

Zweckmäßigerweise umfasst die Chromatographievorrichtung zumindest eine Einrichtung zur Ermittlung einer Stoffeigenschaft des Raffinats und/oder des Extrakts, ggf. ferner zur Ermittlung einer Stoffeigenschaft des Stoffgemischs und/oder des Eluenten, wobei insbesondere ein Gehalt eines Inhaltsstoffs des Raffinats, des Extrakts, des Stoffgemischs und/oder des Eluenten bestimmt wird.

Zweckmäßigerweise werden die Flüsse der Stoffmischung, des Eluenten, des Raffinats und/oder des Extrakts in der Vorrichtung in Abhängigkeit von einem Ergebnis einer Bestimmung der Stoffeigenschaft mittels des Sensors eingestellt, besonders bevorzugt geregelt.

In einer Ausgestaltung der Erfindung wird derart gesteuert und/oder geregelt, dass der Gehalt an dem ersten Stoff im Raffinat maximiert wird und vorzugsweise der Gehalt an dem zweiten und/oder dem dritten Stoff im Raffinat minimiert wird.

In einer Ausführungsform der Erfindung ist oder umfasst die Stoffmischung eine Fettsäuremischung, vorzugweise eine Mischung von ungesättigten, insbesondere mehrfach ungesättigten, Fettsäuren. Besonders bevorzugt enthält die Fettsäuremischung Omega-3-Fettsäuren.

Der erste, aus der Fettsäuremischung zu trennende Stoff ist zweckmäßigerweise eine mehrfach ungesättigte Fettsäure, vorzugsweise eine Omega-3-Fettsäure, besonders bevorzugt Eicosapentaensäure (EPA) und/oder Docosahexaensäure (DHA). Wird EPA als der erste Stoff aus der Stoffmischung getrennt, können DHA und/oder Arachidonsäure (ARA) den zweiten Stoff, der bei der Chromatographie einer größeren Retention unterliegt, bilden. Den dritten Stoff, der bei Chromatographie einer größeren Retention unterliegt als das EPA und als Extrakt entnommen wird, kann Stearidonsäure (SDA) sein. Ziel der Entnahme der Omega-3-Fettsäure, insbesondere der Eicosapentaensäure (EPA) und/oder der Docosahexaensäure (DHA), kann sein, ein Produkt zu erzeugen, dass die jeweilige Omega-3-Fettsäure in besonders hoher Konzentration enthält.

Ferner könnte das Verfahren mit dem Ziel durchgeführt werden, einen Gehalt eines bestimmten Stoffes in der Stoffmischung zu reduzieren. Beispielsweise könnte mittels des Verfahrens Arachidonsäure als der erste Stoff und damit als Raffinat aus einer Omega-3-Fettsäure entnommen werden, um den Gehalt an Arachidonsäure zu reduzieren. Das Zielprodukt würde dann als Extrakt entnommen.

In einer weiteren Ausführungsform der Erfindung ist oder umfasst die Stoffmischung eine Carbonsäuremischung, vorzugsweise eine Cannabinoide enthaltende Mischung. Der erste Stoff ist zweckmäßiger Cannabidiol (CBD). Alternativ könnte Tetrahydrocannabinol (THC), insbesondere Δ9-THC und/oder Δ8-THC, den ersten Stoff bilden. Dies erweist sich als vorteilhaft, wenn aus einer Carbonsäuremischung, insbesondere aus einem Cannabinoid, das THC entfernt werden soll.

Wird CBD als der erste Stoff aus der Carbonsäure getrennt, kann Cannabidivarin (CBDV), Tetrahydrocannabivarin (THCV) und/oder Cannabidivarinsäure (CBDVA) im zweiten Stoff bilden. Der dritte Stoff kann Cannabigerol (CBG), Tetrahydrocannabivarinsäure (THCBA), Δ9- und/oder Δ8-THC, und/oder Cannibigerolsäure (CBGA) gebildet sein.

In einer weiteren Ausgestaltung der Erfindung ist oder umfasst die Stoffmischung eine Mischung aus pre-resolving mediators (PRM), vorzugsweise 18-HEPE, 17-HDHA und/oder 14-HDHA, und/oder specialized pre-resolving mediator (SPM), vorzugsweise Lipoxine, Resolvine, Protectine und/oder Maresine.

In einer weiteren Ausführungsform der Erfindung ist der erste Stoff ein Metabolit einer mehrfach ungesättigten Fettsäure, vorzugsweise von Eicosapentaensäure (EPA) und/oder von Docosahexaensäure (DHA) und/oder von Docosapentaensäure (DPA) oder dieselbe chemische Zusammensatzung aufweist wie der Metabolit.

In einer Ausführungsform der Erfindung wird das Verfahren zweckmäßigerweise durchgeführt wird derart, dass zumindest ein pre-resolving mediator (PRM) und/oder ein specialized pre-resolving mediator (SPM), der bzw. die vorzugsweise von EPA, DHA oder/und von DPA abgeleitet ist bzw. sind, aus der Stoffmischung getrennt wird.

Der pre-resolving mediator (PRM) ist vorzugsweise zumindest ein Stoff aus der Gruppe der Stoffe 18-HEPE, 17-HDHA, 14-HDHA.

Der specialized pre-resolving mediator (SPM)) ist vorzugsweise zumindest ein Stoff aus der Gruppe der Stoffe Lipoxin, Resolvin, Protectin, Maresin.

Das Lipoxin ist bevorzugt zumindest ein Stoff aus der Gruppe der Stoffe LxA4 (5S,6R,15S-trihydroxy-7E,9E,11Z,13E-ETE), LxB4 (5S,14R,15S-trihydroxy-6E,8Z,10E,12E-ETE), 15-epi-LxA4 (5S,6R,15R-trihydroxy-7E,9E,11Z,13E-eicosatetraenoic acid), 15-epi-LxB4 (5S,14R,15R-trihydroxy-6E,8Z,10E,1 2E-eicosatrienoic acid).

Das Resolvin ist zweckmäßigerweise von EPA, DHA oder/und von DPA abgeleitet. Das Resolvin ist bevorzugt zumindest
- ein Stoff aus der Gruppe der Stoffe RvE1 (5S,12R,18R-trihydroxy-6Z,8E,10E,14Z,16E-EPA), 18S-RvE1 (5S,12R,18S-trihydroxy-6Z,8E,10E,14Z,16E-EPA), RvE2 (5S,18R-dihydroxy-6E,8Z,11Z,14Z,16E-EPA), RvE3 (17R,18R/S-dihydroxy-5Z,8Z,11Z,13E,15E-EPA), und/oder
- ein Stoff aus der Gruppe der Stoffe RvD1(7S,8R,17S-trihydroxy-4Z,9E,11E,13Z,15E,1 9Z-DHA), RvD2 (7S,16R,117S-trihydroxy-4Z,8E,10Z,12E,14E,19Z-DHA), RvD3 (4S,11R,17S-trihydroxy-5Z,7E,9E,13Z,15E,19Z-DHA), RvD4 (4S,5R,17S-trihydroxy-6E,8E,10Z,13Z,15E,19Z-DHA), RvD5 (7S,17S-dihydroxy-4Z,8E,10Z,13Z,15E,19Z-DHA), RvD6 (4S,17S-dihydroxy-5E,7Z,10Z,13Z,15E,19Z-DHA),
   und/oder
- ein Stoff aus der Gruppe der Stoffe RvT1 (7,13R,20-trihydroxy-8E,10Z,14E,16Z,18E-DPA), RvT2 (7,8,13R-trihydroxy-9E,11E,14E,16Z,19Z-DPA), RvT3 (7,12,13R-trihydroxy-8Z,10E,14E,16Z,19Z-DPA), RvT4 (7,13R-dihydroxy-8E,10Z,14E,16Z,19Z-DPA).

Das Protectin ist zweckmäßigerweise von DHA oder/und von DPA abgeleitet.

Das Protectin ist bevorzugt zumindest
- ein Stoff aus der Gruppe der Stoffe PD1 bzw. NPD1 (10R,17S-dihydroxy-4Z,7Z,11E,13E,15Z,19Z-DHA), PDX (10S,17S-dihydroxy-4Z,7Z,11E,13Z,15E,19Z-DHA), 22-hydroxy-PD1 (10R,17S,22-trihydroxy-4Z,7Z,11E,13E,15Z,19Z-DHA), 17-epi-PD1 bzw. AT-PD1 (10R,17R-dihydroxy-4Z,7Z,11E,13E,15Z,19Z-DHA), 10-epi-PD1 bzw. ent-AT-NPD1 (10S,17S-Dihydroxy-4Z,7Z,11E,13E,15Z,19Z-DHA)
   und/oder
- ein Stoff aus der Gruppe der Stoffe PD1n-3 (10,17-dihydroxy-7,11,13,15,19-DPA), PD2n-3 (16,17-dihydroxy-7,10,12,14,19-DPA).

Das Maresin ist zweckmäßigerweise von DHA oder/und von DPA abgeleitet.

Das Maresin ist bevorzugt zumindest
- ein Stoff aus der Gruppe der Stoffe MaR1 (7R,14S-dihydroxy-4Z,8E,10E,12Z,16Z,19Z-DHA), MaR2 (13R,14S-dihydroxy-4Z,7Z,9E,11E,16Z,19Z-DHA), 7-epi-MaR1 (7S,14S-dihydroxy-4Z,8E,10Z,12E,16Z,19Z-DHA), MaR-L1 (14S,22-dihydroxy-4Z,7Z,10Z,12E,16Z,19Z-DHA), MaR-L2 (14R,22-dihydroxy-4Z,7Z,10Z,12E,16Z,19Z-DHA),
   und/oder
- ein Stoff aus der Gruppe der Stoffe MaR1n-3 (7S,14S-dihydroxy-8E,10E,12Z,16Z,19Z-DPA), MaR2n-3 (13,14-dihydroxy-7,9,111,16,19-DPA), MaR3n-3 (13,14-dihydroxy-7,9,111,16,19-DPA).

Die Erfindung betrifft ferner eine Vorrichtung, mit der sich das beschriebene Trennverfahren durchführen lässt. Die Vorrichtung umfasst neben den genannten Chromatographiesäulen, den genannten Zuleitungs- und/oder Ableitungseinrichtungen vorzugsweise eine Einrichtung zur Bestimmung einer Stoffeigenschaft des Raffinats und/oder des Extrakts, insbesondere zur Bestimmung eines Gehalts eines Inhaltsstoffs. Mittels der Detektionseinrichtung werden vorzugweise physikalische Eigenschaften, insbesondere Lichtabsorption, Fluoreszenz, Lichtstreuung und/oder Wärmeleitfähigkeit, und/oder chemische Eigenschaften, beispielsweise Färbung oder dergleichen, ermittelt und dadurch der Stoffgehalt bestimmt.

Die Vorrichtung umfasst vorzugsweise ferner eine Einrichtung zur Einstellung eines Stoffflusses, insbesondere eines Zu- und/oder Abflusses, der Stoffmischung, des Eluenten, des Raffinats und/oder des Extrakts.

In einer besonders bevorzugten Ausführungsform der Erfindung weist die Vorrichtung eine Einrichtung zur Regelung und/oder Steuerung von Stoffflüssen, insbesondere Zu- und/oder Abflüssen, über die Zu- und/oder Ableitungseinrichtungen in Abhängigkeit von einem Ergebnis der Bestimmung mittels der Detektionseinrichtung auf. Vorzugsweise ist die Regelungs- und/oder Steuerungseinrichtung dazu vorgesehen, die jeweiligen Stoffflüsse derart einzustellen, dass zumindest eines des obengenannten Gehaltsverhältnisse im Raffinat bzw. Extrakt erreicht wird.

Die Erfindung betrifft ferner ein Computerprogrammprodukt, mittels dessen sich das erfindungsgemäße Verfahren durchführen lässt. Das Computerprogrammprodukt kann zweckmäßigerweise direkt in den internen Speicher eines digitalen Computers geladen werden und umfasst Softwareabschnitte, mit denen, wenn das Computerprogramm auf einem Computer läuft, Schritte des erfindungsgemäßen Verfahrens durchgeführt werden können, durchführen. Das Computerprogrammprodukt weist vorzugsweise Schnittstellen zum Zusammenwirken mit einer erfindungsgemäßen Vorrichtung auf. Das Computerprogrammprodukt kann die genannte Steuerungs- und/oder Regelungseinrichtung bilden oder Teil davon sein. Vorzugsweise ist das Computerprogrammprodukt, insbesondere aufgrund der Schnittstellen, dazu vorgesehen, Ergebnis der Bestimmung mittels der Detektionseinrichtung zu verarbeiten und die Zu- und/oder Ableitungseinrichtungen einzustellen.

Das genannte Computerprogrammprodukt ist zweckmäßigerweise dazu vorgesehen, einzelne, mehrere oder sämtliche der Verfahrensschritte auszuführen, wenn es auf dem Computer läuft.

Das Computerprogramm ist vorzugsweise ein auf einen, flüchtigen oder nichtflüchtigen Datenträger, vorzugsweise RAM, ROM, DRAM, SRAM, EPROM, EEPROM oder dergleichen, oder einem Gerät, insbesondere einem Personalcomputer, einem Gerät mit eingebettetem Prozessor gespeichertes Computerprogrammprodukt oder eine für eine Übersendung über ein Rechnernetzwerk, insbesondere das Internet, geeignete, Daten darstellende Signalfolge.

Die Erfindung betrifft ferner ein Datenträgersignal, das das genannte Computerprodukt überträgt.

Die Erfindung wird nachfolgend anhand von Ausführungsbeispielen und den beigefügten Zeichnungen, die sich auf die Ausführungsbeispiele beziehen, näher erläutert. Es zeigen:
- Fig. 1: schematisch eine erfindungsgemäße Vorrichtung,
- Fig. 2: die Vorrichtung nach Fig. 1 in verschiedenen Stellungen und
- Fig. 3: eine schematische Grafik zu Retentionszeiten und
- Fig. 4 und 5: schematisch Grafiken mit Retentionszeiten von Stoffen verschiedener Stoffmischungen.

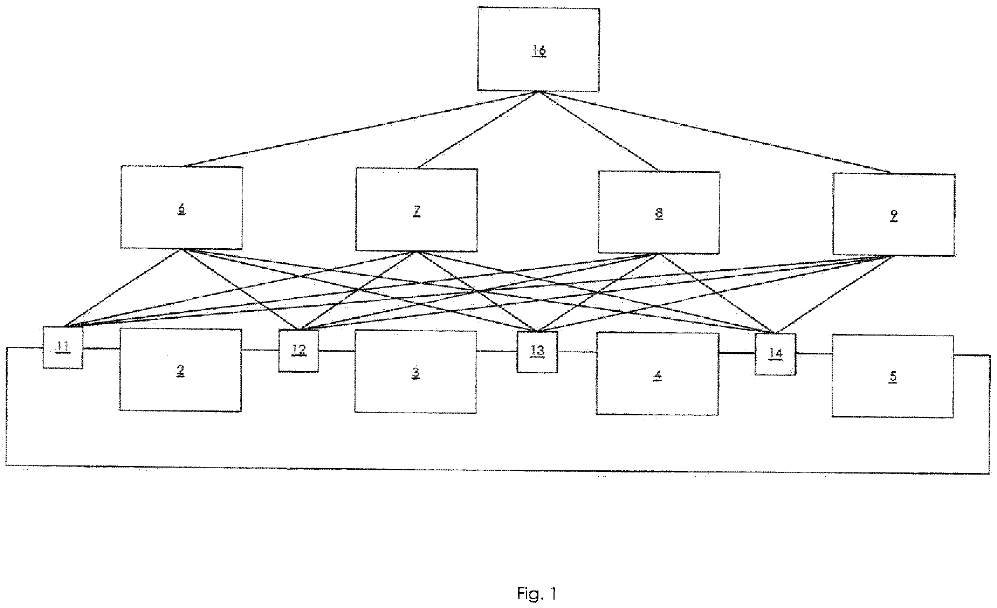

In Fig. 1 ist schematisch eine erfindungsgemäße Chromatographievorrichtung 1 dargestellt, die Chromatographiesäulen 2,3,4,5 umfasst, die untereinander derart verbunden sind, dass sie in Strömungsverbindung zueinander stehen. Zwischen den Chromatographiesäulen sind Anschlüsse 11, 12, 13, 14 angeordnet. Jeder der Anschlüsse 11, 12, 13, 14 ist mit einer Einrichtung 6 zur Zuleitung eines Eluenten, einer Einrichtung 7 zur Ableitung eines Extrakts, einer Einrichtung 8 zur Zuleitung eines Stoffgemischs und einer Einrichtung 9 zur Ableitung eines Raffinats verbunden. Die genannten Einrichtungen 6,7,8,9 weisen jeweilig Ventile auf, mittels derer sich Stoffflüsse, insbesondere Zu- oder/und Abflüsse des Eluenten, des Extrakts, des Stoffgemischs bzw. des Raffinates einstellen lassen, insbesondere die Größe der jeweiligen Flüsse.

Die Vorrichtung umfasst einen Sensor zur Bestimmung eines Gehalts eines ersten Stoffes in dem Extrakt und einen Sensor zur Bestimmung eines Gehalts des ersten Stoffes in dem Raffinat.

Ferner weist die Vorrichtung 1 eine Einrichtung 16 zur Regelung und/oder Steuerung der genannten Zu- und/oder Abflüsse, insbesondere durch Einstellung der genannten Ventile, auf. Die Regelungs- und/oder Steuerungseinrichtung 16 umfasst vorzugsweise einen eingebetteten Prozessor, auf dem ein Computerprogramm gespeichert ist, das dazu vorgesehen ist, unter Verarbeitung von durch die Sensoren bereitgestellten Daten die Regelung und/oder Steuerung durchzuführen.

Je nach Offen- bzw. Schließposition der verschiedenen Ventile können die Positionen, an welche der Eluent bzw. das Stoffgemisch zugeführt werden und das Extrakt bzw. das Raffinat abgeführt werden, eingestellt werden. Dementsprechend lassen sich in den verschiedenen Chromatographiesäulen 2,3,4,5 wechselnd Zonen I,II,III,IV bilden, wobei
a) eine erste Zone I jeweilig zwischen einem Anschluss, über welchen der Eluent zugeleitet wird, und einen Anschluss, über welchen der Extrakt abgeleitet wird,
b) eine zweite Zone II zwischen einem Anschluss, über den der Extrakt abgeleitet wird, und einem Anschluss, über den das Stoffgemisch zugeleitet wird,
c) eine dritte Zone III zwischen einem Anschluss, über den das Stoffgemisch zugeleitet wird und einem Anschluss, über den das Raffinat abgeleitet wird, und
d) eine vierte Zone IV zwischen einem Anschluss, über den das Raffinat abgeleitet wird, und einem Anschluss, über den der Eluent zugeleitet wird, gebildet wird.

Die Steuerungs- und/oder Regelungseinrichtung 16 ist dazu eingerichtet, in Abhängigkeit von Signalen der Sensoren 16, Positionen der Ventile einzustellen, um Stoffflüsse, insbesondere Zu- und/oder Abflüsse, einzustellen. Durch Verstellung der verschiedenen Ventile lassen sich die Zonen I,II,III,IV unterschiedlich in der Chromatographiesäulen 2,3,4,5 zuordnen. Die Einstellung der Ventilpositionen erfolgt derart, dass der erste Stoff als das Raffinat entnommen werden kann. Fig. 2a zeigt die erfindungsgemäße Vorrichtung 1 in einer Ventilschaltposition, in welcher die Chromatographiesäule 2 die Zone I, die Chromatographiesäule 3 die Zone II, die Chromatographiesäule 4 die Zone III und die Chromatographiesäule 5 die Zone IV bildet.

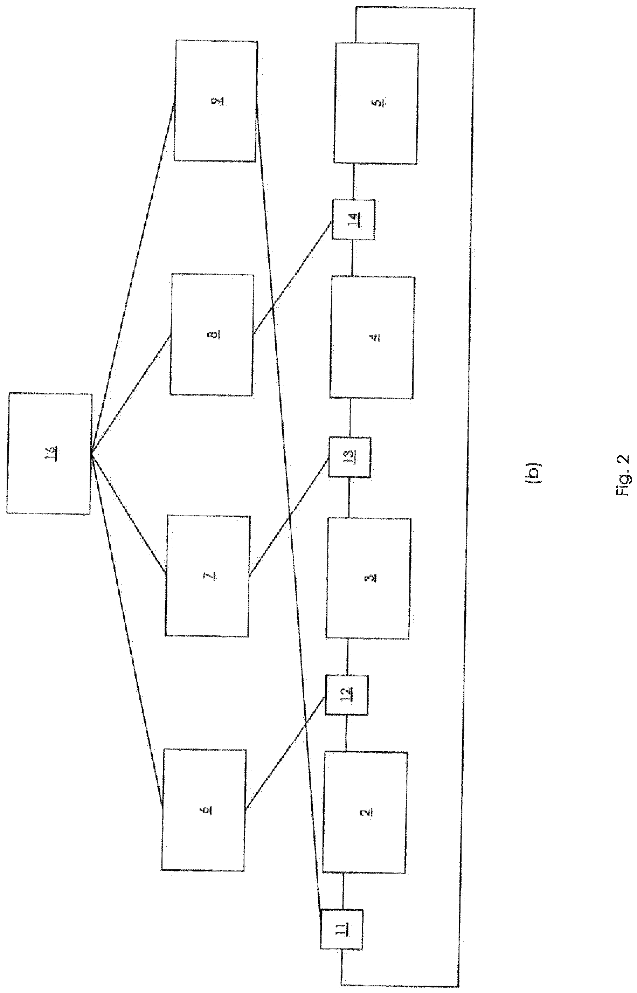

Durch Umschalten der Ventile können die Zonen I,II,III,IV in jeweilig anderen Chromatographiesäulen 2,3,4,5 gebildet werden, beispielsweise kann bei entsprechender Ventilschaltung, so wie in Fig. 2b gezeigt, die Chromatographiesäule 2 die Zone IV, die Chromatographiesäule 3 die Zone I, die Chromatographiesäule 4 die Zone II und die Chromatographiesäule 5 die Zone III bilden. Die Umschaltung der Ventile erfolgt im Wesentlichen wie nach den bekannten TMB bzw. SMB-Chromatographieverfahren bekannt, allerdings unter Berücksichtigung der unten erläuterten Voraussetzungen.

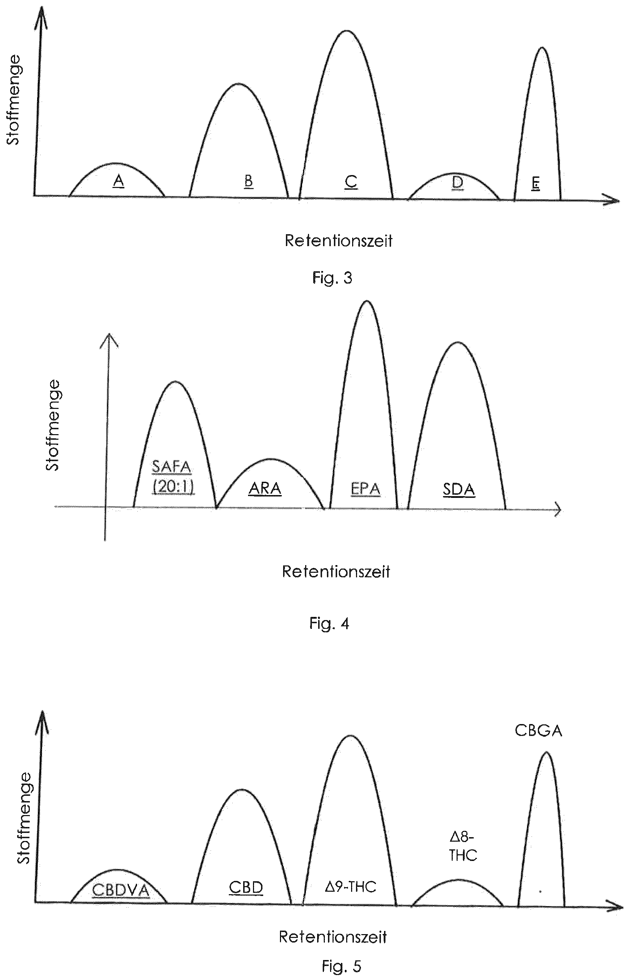

Die Durchführung des erfindungsgemäßen Verfahrens wird näher anhand der Fig. 3 erläutert, die Retentionszeiten unterschiedlicher Stoffe A,B,C,D und E eines Stoffgemischs zeigt. Aus dem Stoffgemisch soll der Stoff D herausgetrennt werden. Der Stoff D weist, wie die Fig. 3 zeigt, eine größere Retentionszeit als die Stoffe A,B,C und eine kleinere Retentionszeit als der Stoff E auf. Um den Stoff D als Raffinat entnehmen zu können, werden die Zu- und Abflüsse derart eingestellt, dass der Stoff D nach Durchfließen der Zone III als Raffinat entnommen werden kann. Die Stoffe A,B,C, die eine kleinere Retentionszeit aufweisen als der Stoff D werden nach Zone I als Extrakt entnommen. Erfindungsgemäß werden darüber hinaus die Zu- und Abflüsse insbesondere derart eingestellt, dass sich in der Zone IV derartiger Fluss bildet, dass der Stoff E in die Zone I gedrückt wird und sich gemeinsam mit den Stoffen A,B,C in Zone I als Extrakt entnehmen lässt.

In einem ersten konkreten Ausführungsbeispiel wird das erfindungsgemäße Trennverfahren an einem Stoffgemisch aus mehrfach ungesättigten Fettsäuren durchgeführt. Das Stoffgemisch, insbesondere ein Öl, beispielsweise ein Fischöl, ein Algenöl oder/und ein Leinöl, weist DHA, ARA, EPA und SDA auf. Die entsprechenden Retentionszeiten sind in Fig. 4 gezeigt. EPA soll als Raffinat entnommen werden. DHA und ARA weisen kleinere Retentionszeiten auf als EPA. SDA dagegen weist eine größere Retentionszeit auf. Während es nach den herkömmlichen Chromatographieverfahren notwendig wäre, zunächst eine Stofftrennung nach einerseits DHA und ARA und anderseits EPA und SDA durchzuführen und anschließend eine weitere Trennung des bereits abgetrennten Teils mit EPA und SDA zur Trennung der beiden Komponenten durchzuführen, werden wie oben erläutert bei erfindungsgemäßer Durchführung des Verfahrens nicht nur DHA und ARA als Extrakt entnommen, sondern darüber hinaus bei entsprechender Einstellung der Zu- und Abflüsse auch SDA als Extrakt entnommen. Die Entnahme des EPA erfolgt ausschließlich als Raffinat.

**Tabelle 1**

| Q_{I} | Q_{II} | Q_{III} | Q_{IV} | Q_{Eluent} | Q_{SG} | Q_{Raf} | Q_{Extr} | Gehalte im Raffinat | | |
|---|---|---|---|---|---|---|---|---|---|---|
| mL/min | mL/min | mL/min | mL/min | mL/min | mL/min | mL/min | mL/min | %SDA | % EPA | % DHA |
| 353 | 263,4 | 265 | 190 | 163 | 1,6 | 75 | 89,6 | 4,10% | 91,7% | 0,0% |
| 353 | 263,4 | 265 | 200 | 153 | 1,6 | 65 | 89,6 | 4,40% | 90,7% | 0,0% |
| 353 | 263,4 | 265 | 210 | 143 | 1,6 | 55 | 89,6 | 4,50% | 91,2% | 0,0% |
| 353 | 263,4 | 265 | 220 | 133 | 1,6 | 45 | 89,6 | 3,70% | 93,2% | 0,0% |
| 353 | 263,4 | 265 | 230 | 123 | 1,6 | 35 | 89,6 | 3,00% | 93,0% | 0,0% |

Die Tabelle 1 zeigt, dass im Raffinat unterschiedliche Gehalte an SDA und EPA erzielt werden können, wenn in der Zone IV unterschiedliche Flüsse Q_{IV} eingestellt werden. Der niedrigste Gehalt an SDA lässt sich erzielen, wenn in der Zone IV ein vergleichsweise großer Fluss eingestellt wird. Die Einstellungen der unterschiedlichen Flüsse in den Zonen I,II,III,IV wird, wie der Tabelle 1 ebenfalls zu entnehmen ist, dadurch erreicht, dass unterschiedliche Stoffflüsse, insbesondere Zu- und/oder Abflüsse, des Eluenten (Q_{Eluent}), des Stoffgemischs (Q_{SG}), des Raffinats (Q_{Raf}) und des Extrakts (Q_{Extr}) eingestellt werden.

In einem weiteren Ausführungsbeispiel ist vorgesehen, aus derselben Stoffmischung, für die in Fig. 4 die Retentionszeiten gezeigt sind, ARA als Raffinat zu entnehmen, um die Stoffmischung von der Arachidonsäure zu befreien. In diesem Fall werden die Stoffflüsse derart eingestellt, dass das EPA und das SDA in die Zone I gedrückt werden, damit sie gemeinsam mit dem DHA als Extrakt entnommen werden können. Es versteht sich, dass dazu in der Zone IV im Vergleich zu der herkömmlichen Anwendung der SMB-Chromatographie größere Durchflüsse vorzusehen sind.

In einem weiteren Ausführungsbeispiel wird das Trennverfahren an einem Stoffgemisch durchgeführt, dass ein Cannabidiol enthält. Fig. 5 zeigt schematisch in einem Diagramm Retentionszeiten einzelner ausgewählter Stoffe des Stoffgemischs, nämlich diejenigen von CBDVA, CBD, Δ9-THC, Δ8-THC und CBGA.

Es wird beabsichtigt, aus dem Stoffgemisch CBD zu gewinnen, hierfür werden die Stoffflüsse bei der Durchführung der Chromatographie derart eingestellt, dass CBD als das einzige Raffinat entnommen werden kann. CBDVA, das eine geringere Retentionszeit aufweist als CBD, sowie Δ9-THC, Δ8-THC und CBGA, die eine größere Retentionszeit aufweisen als CBD, werden gemeinsam als Extrakt entnommen.

Ferner ist es möglich, mittels des erfindungsgemäßen Verfahrens die letztgenannte Cannabidiolstoffmischung von THC zu befreien, indem die Stoffflüsse derart eingestellt werden, dass Δ9-THC und Δ8-THC gemeinsam als Raffinat entnommen werden. CBDVA und CBD, die eine geringere Retentionszeit als Δ9-THC und Δ8-THC aufweisen, und CBGA, das eine größere Retentionszeit als Δ9-THC und Δ8-THC aufweist, werden gemeinsam als Extrakt entnommen.

## Patentansprüche

1. Verfahren zum Trennen eines ersten Stoffes aus einer Stoffmischung mittels Chromatographie, wobei die Stoffmischung zumindest einen zweiten Stoff aufweist, der bei der Chromatographie einer größeren Retention unterliegt als der erste Stoff, und zumindest einen dritten Stoff aufweist, der bei der Chromatographie einer geringeren Retention unterliegt als der erste Stoff,
wobei die Stoffmischung einer Vorrichtung (1) zur Chromatographie zugeführt wird, die mehrere miteinander verbundene Chromatographiesäulen (2,3,4,5) umfasst, der Vorrichtung (1) die Stoffmischung und ein Eluent zugeführt werden sowie der zweite Stoff als Extrakt und der erste Stoff als Raffinat entnommen werden,
**dadurch gekennzeichnet,**
**dass** der dritte Stoff als Extrakt entnommen wird.

2. Verfahren nach Anspruch 1,
**dadurch gekennzeichnet,**
**dass** der dritte Stoff und der zweite Stoff gemeinsam als Extrakt entnommen werden.

3. Verfahren nach Anspruch 1 oder 2,
**dadurch gekennzeichnet,**
**dass** die Chromatographiesäulen (2,3,4,5) Zonen (I,II,III,IV) bilden, wobei
a) eine erste Zone (I) zwischen einer Einrichtung (6) zur Zuleitung des Eluenten und einer Einrichtung (7) zur Ableitung des Extrakts,
b) eine zweite Zone (II) zwischen einer Einrichtung (7) zur Ableitung des Extrakts und einer Einrichtung (8) zur Zuleitung des Stoffgemischs,
c) eine dritte Zone (III) zwischen einer Einrichtung (8) zur Zuleitung Stoffgemischs und einer Einrichtung (9) zur Ableitung des Raffinates und
d) eine vierte Zone (IV) zwischen einer Einrichtung (9) zur Ableitung des Raffinates und einer Einrichtung (6) zur Zuleitung des Eluenten gebildet ist.

4. Verfahren nach Anspruch 3,
**dadurch gekennzeichnet,**
**dass** Stoffflüsse, insbesondere Zu- und/oder Abflüsse, über die Zu- und/oder Ableitungseinrichtungen (6,7,8,9) derart eingestellt werden, dass in einer Zone (IV) zwischen einer Einrichtung (9) zur Ableitung des Raffinates und einer Einrichtung (6) zur Zuleitung des Eluenten ein derart großer Fluss vorliegt, dass der dritte Stoff als Extrakt entnommen werden kann.

5. Verfahren nach einem der Ansprüche 1 bis 4,
**dadurch gekennzeichnet,**
**dass** die Einrichtung (6) zur Zuleitung des Eluenten, die Einrichtung (7) zur Ableitung des Extrakts, die Einrichtung (8) zur Zuleitung des Stoffgemischs und die Einrichtung (9) zur Ableitung des Raffinates mehrere Zuleitungs- bzw. Ableitungsanschlüsse (10,11,12,13) zwischen verschiedenen der Chromatographiesäulen (2,3,4,5) aufweisen und jeweilige Stoffflüsse durch die Zuleitungs- bzw. Ableitungsanschlüsse (10,11,12,13) derart eingestellt werden, dass die Zonen (I,II,III,IV) in der Vorrichtung (1) wechselnd in unterschiedlichen der Chromatographiesäulen (2,3,4,5) gebildet werden.

6. Verfahren nach einem der Ansprüche 1 bis 5,
**dadurch gekennzeichnet,**
**dass** die Stoffmischung eine Fettsäuremischung, vorzugsweise eine Mischung von ungesättigten, insbesondere mehrfach ungesättigten, Fettsäuren, und/oder eine Carbonsäuremischung, vorzugsweise eine Mischung von Cannabinoiden, und/oder eine Mischung von pre-resolving mediators (PRM), vorzugsweise 18-HEPE, 17-HDHA und/oder 14-HDHA, und/oder von specialized pre-resolving mediators (SPM), vorzugsweise Lipoxine, Resolvine, Protectine und/oder Maresine, ist oder umfasst.

7. Verfahren nach einem der Ansprüche 1 bis 6,
**dadurch gekennzeichnet,**
**dass** der erste Stoff eine mehrfach ungesättigte Fettsäure, vorzugsweise Eicosapentaensäure (EPA) und/oder Docosahexaensäure (DHA), oder Cannabidiol (CBD) oder Tetrahydrocannabinol (THC) ist.

8. Verfahren nach einem der Ansprüche 1 bis 7,
**dadurch gekennzeichnet,**
**dass**
a) der dritte Stoff Stearidonsäure (SDA) und/oder der zweite Stoff Arachdionsäure (ARA) und/oder Docosahexaensäure (DHA) ist,
b) der dritte Stoff Stearidonsäure (SDA) und/oder Eicosapentaensäure (EPA) und/oder der zweite Stoff Arachdionsäure (ARA)
oder
c) der dritte Stoff Tetrahydrocannabinol (THC), CBGA und/oder CBG ist und/oder der zweite Stoff CBDVA, THCV und/oder CBDV ist.

9. Verfahren nach einem der Ansprüche 1 bis 8,
**dadurch gekennzeichnet,**
**dass** der erste Stoff ein Metabolit einer mehrfach ungesättigten Fettsäure, vorzugsweise von Eicosapentaensäure (EPA) und/oder von Docosahexaensäure (DHA) und/oder von Docosapentaensäure (DPA), ist oder dieselbe Zusammensatzung aufweist wie der Metabolit.

10. Verfahren nach einem der Ansprüche 1 bis 9,
**dadurch gekennzeichnet,**
**dass** zumindest ein pre-resolving mediator (PRM), vorzugsweise 18-HEPE, 17-HDHA und/oder 14-HDHA, und/oder zumindest ein specialized pre-resolving mediator (SPM), vorzugsweise Lipoxine, Resolvine, Protectine und/oder Maresine, aus der Stoffmischung entnommen wird.

11. Verfahren nach einem der Ansprüche 1 bis 10,
**dadurch gekennzeichnet,**
**dass** der erste Stoff ein pre-resolving mediator (PRM) und/oder ein specialized pre-resolving mediator (SPM) ist.

12. Verfahren nach einem der Ansprüche 1 bis 11,
**dadurch gekennzeichnet,**
**dass**
a) der dritte Stoff ein pre-resolving mediator (PRM) und/oder ein specialized pre-resolving mediator (SPM) und/oder der zweite Stoff Arachdionsäure (ARA) und/oder Docosahexaensäure (DHA) ist,
b) der dritte Stoff ein pre-resolving mediator (PRM) und/oder ein specialized pre-resolving mediator (SPM) und/oder der zweite Stoff Eicosapentaensäure (EPA) und/oder Arachdionsäure (ARA)
oder
c) der dritte Stoff ein pre-resolving mediator (PRM) und/oder ein specialized pre-resolving mediator (SPM) und/oder der zweite Stoff Docosapentaensäure (DPA).

13. Vorrichtung zum Trennen eines ersten Stoffes aus einer Stoffmischung mittels Chromatographie, die mehrere miteinander verbundene Chromatographiesäulen (2,3,4,5) umfasst und eine Einrichtung zur Zuleitung der Stoffmischung, eine Einrichtung zur Zuleitung eines Eluenten, eine Einrichtung zur Ableitung eines Extrakten sowie eine Einrichtung zur Ableitung eines Raffinates aufweist, wobei die Stoffmischung zumindest einen zweiten Stoff aufweist, der bei der Chromatographie einer größeren Retention unterliegt als der erste Stoff, und zumindest einen dritten Stoff umfasst, der bei der Chromatographie einer geringeren Retention unterliegt als der erste Stoff, wobei die Vorrichtung dazu eingerichtet ist, den zweiten Stoff als Extrakt und den ersten Stoff als Raffinat zu entnehmen,
**dadurch gekennzeichnet,**
**dass** die Vorrichtung dazu eingerichtet ist, den dritten Stoff als Extrakt zu entnehmen.

14. Vorrichtung nach Anspruch 13,
**gekennzeichnet durch** eine Einrichtung zur Einstellung eines Zu- und/oder Abflusses der Stoffmischung, des Eluenten, des Raffinates und/oder des Extrakts derart, dass der dritte Stoff gemeinsam mit dem zweiten Stoff als Extrakt entnommen werden kann.

15. Computerprogrammprodukt, das direkt in den internen Speicher eines digitalen Computers geladen werden kann und Softwareabschnitte umfasst, mit denen, wenn das Computerprogrammprodukt auf einem Computer läuft, Schritte eines Verfahrens zum Steuern und/oder Regeln einer Stofftrennvorrichtung ausgeführt werden, wobei die Stofftrennvorrichtung mehrere miteinander verbundene Chromatographiesäulen (2,3,4,5) umfasst und zum Trennen eines ersten Stoffes aus einer Stoffmischung vorgesehen ist, wobei die Stoffmischung zumindest einen zweiten Stoff aufweist, der bei Durchführung einer Chromatographie einer größeren Retention unterliegt als der erste Stoff, und einen dritten Stoff aufweist, der bei der Chromatographie einer geringeren Retention unterliegt als der erste Stoff, wobei der Vorrichtung (1) die Stoffmischung und ein Eluent zugeführt werden sowie der zweite Stoff als Extrakt und der erste Stoff als Raffinat entnommen werden,
**dadurch gekennzeichnet,**
**dass** Stoffflüsse, insbesondere Zu- und/oder Abflüsse, der Stoffmischung, des Eluenten, des Raffinates und/oder des Extrakts in der Vorrichtung (1) derart eingestellt werden, dass der dritte Stoff als Extrakt entnommen wird.

## Claims

1. Method for separating a first substance from a substance mixture by chromatography, wherein the substance mixture comprises at least one second substance which is subject to greater retention than the first substance in the chromatography, and comprises at least one third substance which is subject to lower retention than the first substance in the chromatography,
wherein the substance mixture is fed to an apparatus (1) for chromatography comprising a plurality of interconnected chromatography columns (2, 3, 4, 5), the substance mixture and an eluent are fed to the apparatus (1), and the second substance is withdrawn as an extract and the first substance is withdrawn as a raffinate,
**characterized**
**in that** the third substance is withdrawn as an extract.

2. Method according to Claim 1,
**characterized**
**in that** the third substance and the second substance are withdrawn together as an extract.

3. Method according to Claim 1 or 2,
**characterized**
**in that** the chromatography columns (2, 3, 4, 5) form zones (I, II, III, IV), wherein
a) a first zone (I) is formed between a device (6) for supplying the eluent and a device (7) for discharging the extract,
b) a second zone (II) is formed between a device (7) for discharging the extract and a device (8) for supplying the substance mixture,
c) a third zone (III) is formed between a device (8) for supplying the substance mixture and a device (9) for discharging the raffinate and
d) a fourth zone (IV) is formed between a device (9) for discharging the raffinate and a device (6) for supplying the eluent.

4. Method according to Claim 3,
**characterized**
**in that** substance flows, in particular inflows and/or outflows, are adjusted via the supply and/or discharge devices (6, 7, 8, 9) in such a way that in a zone (IV) between a device (9) for discharging the raffinate and a device (6) for supplying the eluent there is such a large flow that the third substance can be withdrawn as an extract.

5. Method according to any of Claims 1 to 4,
**characterized**
**in that** the device (6) for supplying the eluent, the device (7) for discharging the extract, the device (8) for supplying the substance mixture and the device (9) for discharging the raffinate comprise a plurality of supply and discharge connections (10, 11, 12, 13) between different chromatography columns (2, 3, 4, 5) and respective substance flows through the supply and discharge connections (10, 11, 12, 13) are adjusted in such a way that the zones (I, II, III, IV) in the apparatus (1) are formed alternately in different chromatography columns (2, 3, 4, 5).

6. Method according to any of Claims 1 to 5,
**characterized**
**in that** the substance mixture is or comprises a fatty acid mixture, preferably a mixture of unsaturated, in particular polyunsaturated, fatty acids, and/or a carboxylic acid mixture, preferably a mixture of cannabinoids, and/or a mixture of pre-resolving mediators (PRM), preferably 18-HEPE, 17-HDHA and/or 14-HDHA, and/or of specialized pre-resolving mediators (SPM), preferably lipoxins, resolvins, protectins and/or maresins.

7. Method according to any of Claims 1 to 6,
**characterized**
**in that** the first substance is a polyunsaturated fatty acid, preferably eicosapentaenoic acid (EPA) and/or docosahexaenoic acid (DHA), or cannabidiol (CBD) or tetrahydrocannabinol (THC).

8. Method according to any of Claims 1 to 7,
**characterized**
**in that**
a) the third substance is stearidonic acid (SDA) and/or the second substance is arachidonic acid (ARA) and/or docosahexaenoic acid (DHA),
b) the third substance is stearidonic acid (SDA) and/or eicosapentaenoic acid (EPA) and/or the second substance is arachidonic acid (ARA)
or
c) the third substance is tetrahydrocannabinol (THC), CBGA and/or CBG and/or the second substance is CBDVA, THCV and/or CBDV.

9. Method according to any of Claims 1 to 8,
**characterized**
**in that** the first substance is a metabolite of a polyunsaturated fatty acid, preferably of eicosapentaenoic acid (EPA) and/or of docosahexaenoic acid (DHA) and/or of docosapentaenoic acid (DPA), or has the same composition as the metabolite.

10. Method according to any of Claims 1 to 9,
**characterized**
**in that** at least one pre-resolving mediator (PRM), preferably 18-HEPE, 17-HDHA and/or 14-HDHA, and/or at least one specialized pre-resolving mediator (SPM), preferably lipoxins, resolvins, protectins and/or maresins, is withdrawn from the substance mixture.

11. Method according to any of Claims 1 to 10,
**characterized**
**in that** the first substance is a pre-resolving mediator (PRM) and/or a specialized pre-resolving mediator (SPM).

12. Method according to any of Claims 1 to 11,
**characterized**
**in that**
a) the third substance is a pre-resolving mediator (PRM) and/or a specialized pre-resolving mediator (SPM) and/or the second substance is arachidonic acid (ARA) and/or docosahexaenoic acid (DHA),
b) the third substance is a pre-resolving mediator (PRM) and/or a specialized pre-resolving mediator (SPM) and/or the second substance is eicosapentaenoic acid (EPA) and/or arachidonic acid (ARA)
or
c) the third substance is a pre-resolving mediator (PRM) and/or a specialized pre-resolving mediator (SPM) and/or the second substance is docosapentaenoic acid (DPA).

13. Apparatus for separating a first substance from a substance mixture by chromatography, comprising a plurality of interconnected chromatography columns (2, 3, 4, 5) and comprising a device for supplying the substance mixture, a device for supplying an eluent, a device for discharging an extract, and a device for discharging a raffinate, wherein the substance mixture comprises at least one second substance which is subject to greater retention than the first substance in the chromatography, and comprises at least one third substance which is subject to lower retention than the first substance in the chromatography, wherein the apparatus is configured to withdraw the second substance as an extract and the first substance as a raffinate,
**characterized**
**in that** the apparatus is configured to withdraw the third substance as an extract.

14. Apparatus according to Claim 13,
**characterized by** a device for adjusting an inflow and/or outflow of the substance mixture, of the eluent, of the raffinate and/or of the extract in such a way that the third substance can be withdrawn together with the second substance as an extract.

15. Computer program product which can be loaded directly into the internal memory of a digital computer and which comprises software sections by means of which, when the computer program product runs on a computer, steps of a method for controlling and/or regulating a substance separation apparatus are carried out, wherein the substance separation apparatus comprises a plurality of interconnected chromatography columns (2, 3, 4, 5) and is provided for separating a first substance from a substance mixture, wherein the substance mixture comprises at least one second substance which is subject to greater retention than the first substance when chromatography is carried out, and comprises a third substance which is subject to lower retention than the first substance in the chromatography, wherein the substance mixture and an eluent are fed to the apparatus (1), and the second substance is withdrawn as an extract and the first substance is withdrawn as a raffinate,
**characterized**
**in that** substance flows, in particular inflows and/or outflows, of the substance mixture, of the eluent, of the raffinate and/or of the extract in the apparatus (1) are adjusted in such a way that the third substance is withdrawn as an extract.

## Revendications

1. Procédé de séparation d'une première substance à partir d'un mélange de substances par chromatographie, le mélange de substances présentant au moins une deuxième substance, qui subit, lors de la chromatographie, une rétention supérieure à celle de la première substance, et au moins une troisième substance, qui subit, lors de la chromatographie, une rétention inférieure à celle de la première substance.
le mélange de substances étant introduit, pour la chromatographie, dans un appareil (1) qui comprend plusieurs colonnes de chromatographie (2, 3, 4, 5) reliées les unes aux autres, l'appareil (1) étant alimenté en mélange de substances et en un éluant et la deuxième substance étant prélevée en tant qu'extrait et la première substance étant prélevée en tant que raffinat, **caractérisé en ce que** la troisième substance est prélevée en tant qu'extrait.

2. Procédé selon la revendication 1, **caractérisé en ce que** la troisième substance et la deuxième substance sont prélevées conjointement en tant qu'extrait.

3. Procédé selon la revendication 1 ou 2, **caractérisé en ce que** les colonnes de chromatographie (2, 3, 4, 5) forment des zones (I, II, III, IV), dans lesquelles
a) une première zone (I) est formée entre un dispositif (6) destiné à introduire l'éluant et un dispositif (7) destiné à évacuer l'extrait,
b) une deuxième zone (I) est formée entre un dispositif (7) destiné à évacuer l'extrait et un dispositif (8) destiné à introduire le mélange de substances,
c) une troisième zone (III) est formée entre un dispositif (8) destiné à introduire le mélange de substances et un dispositif (9) destiné à évacuer le raffinat et
d) une quatrième zone (I) est formée entre un dispositif (9) destiné à évacuer l'extrait et un dispositif (6) destiné à introduire l'éluant.

4. Procédé selon la revendication 3, **caractérisé en ce que** les flux de substances, en particulier les flux d'alimentation et/ou d'évacuation par l'intermédiaire des dispositifs d'alimentation et/ou d'évacuation (6, 7, 8, 9), sont réglés de telle sorte qu'il existe, dans une zone (IV) entre un dispositif (9) destiné à évacuer le raffinat et un dispositif (6) destiné à introduire l'éluant, un flux d'une grandeur telle que la troisième substance peut être prélevée en tant qu'extrait.

5. Procédé selon l'une des revendications 1 à 4, **caractérisé en ce que** le dispositif (6) destiné à introduire l'éluant, le dispositif (7) destiné à évacuer l'extrait, le dispositif (8) destiné à introduire le mélange de substances et le dispositif (9) destiné à évacuer le raffinat présentent plusieurs raccords d'alimentation ou, selon le cas d'évacuation (10, 11, 12, 13) entre différentes colonnes parmi les colonnes chromatographie (2, 3, 4, 5) et les différents flux de substances à travers les raccords d'alimentation ou, selon le cas, d'évacuation (10, 11, 12, 13) sont réglés de telle sorte que les zones (I, II, III, IV) dans l'appareil (1) sont formées en alternance dans différentes colonnes parmi les colonnes de chromatographie (2, 3, 4, 5).

6. Procédé selon l'une des revendications 1 à 5, **caractérisé en ce que** le mélange de substances est ou comprend un mélange d'acides gras, de préférence un mélange d'acides gras insaturés, en particulier polyinsaturés, et/ou un mélange d'acides carboxyliques, de préférence un mélange de cannabinoïdes, et/ou un mélange de médiateurs pro-résolution (PRM), de préférence le 18-HEPE, le 17-HDHA et/ou le 14-HDHA et/ou de médiateurs pro-résolution spécialisés (SPM), de préférence la lipoxine, la résolvine, la protectine et/ou la marésine.

7. Procédé selon l'une des revendications 1 à 6, **caractérisé en ce que** la première substance est un acide gras polyinsaturé, de préférence l'acide eicosapentaénoïque (EPA) et/ou l'acide docosahexaénoïque (DHA), ou le cannabidiol (CBD) ou le tétrahydrocannabinol (THC).

8. Procédé selon l'une des revendications 1 à 7, **caractérisé en ce que**
a) la troisième substance est l'acide stéaridonique (SDA) et/ou la deuxième substance est l'acide arachidonique (ARA) et/ou l'acide docosahexaénoïque (DHA),
b) la troisième substance est l'acide stéaridonique (SDA) et/ou l'acide eicosapentaénoïque (EPA) et/ou la deuxième substance est l'acide arachidonique (ARA) ou
c) la troisième substance est le tétrahydrocannabinol (THC), le CBGA et/ou le CBG et/ou la deuxième substance est le CBDVA, la THCV et/ou la CBDV.

9. Procédé selon l'une des revendications 1 à 8, **caractérisé en ce que** la première substance est un métabolite d'un acide gras polyinsaturé, de préférence de l'acide eicosapentaénoïque (EPA) et/ou de l'acide docosahexaénoïque (DHA) et/ou de l'acide docosapentaénoïque (DPA), ou présente la même composition que celle du métabolite.

10. Procédé selon l'une des revendications 1 à 9, **caractérisé en ce qu'**au moins un médiateur pro-résolution (PRM), de préférence le 18-HEPE, le 17-HDHA et/ou 14-HDHA, et/ou au moins un médiateur pro-résolution spécialisé (SPM), de préférence la lipoxine, la résolvine, la protectine et/ou la marésine, est/sont prélevé(s) du mélange de substances.

11. Procédé selon l'une des revendications 1 à 10, **caractérisé en ce que** la première substance est un médiateur pro-résolution (PRM) et/ou un médiateur pro-résolution spécialisé (SPM).

12. Procédé selon l'une des revendications 1 à 11, **caractérisé en ce que**
a) la troisième substance est un médiateur pro-résolution (PRM) et/ou un médiateur pro-résolution spécialisé (SPM) et/ou la deuxième substance est l'acide arachidonique (ARA) et/ou l'acide docosahexaénoïque (DHA),
b) la troisième substance est un médiateur pro-résolution (PRM) et/ou un médiateur pro-résolution spécialisé (SPM) et/ou la deuxième substance est l'acide eicosapentaénoïque (EPA) et/ou l'acide arachidonique (ARA) ou
c) la troisième substance est un médiateur pro-résolution (PRM) et/ou un médiateur pro-résolution spécialisé (SPM) et/ou la deuxième substance est l'acide docosapentaénoïque (DPA).

13. Appareil pour la séparation d'une première substance à partir d'un mélange de substances au moyen d'une chromatographie, lequel appareil comprend plusieurs colonnes de chromatographie (2, 3, 4, 5) reliées les unes aux autres et un dispositif destiné à introduire le mélange de substances, un dispositif destiné à introduire un éluant, un dispositif destiné à évacuer un extrait ainsi qu'un dispositif destiné à évacuer un raffinat, le mélange de substances présentant au moins une deuxième substance, qui subit, lors de la chromatographie, une rétention supérieure à celle de la première substance, et au moins une troisième substance, qui subit, lors de la chromatographie, une rétention inférieure à celle de la première substance, l'appareil étant conçu pour prélever la deuxième substance en tant qu'extrait et la première substance en tant que raffinat, **caractérisé en ce que** l'appareil est conçu pour prélever la troisième substance en tant qu'extrait.

14. Appareil selon la revendication 13, **caractérisé par** un dispositif destiné à régler un flux d'alimentation et/ou d'évacuation du mélange de substances, de l'éluant, du raffinat et/ou de l'extrait de telle sorte que la troisième substance peut être prélevée conjointement avec la deuxième substance en tant qu'extrait.

15. Produit-programme informatique qui peut être chargé directement dans la mémoire interne d'un ordinateur numérique et qui comprend des sections logicielles avec lesquelles, lorsque le produit-programme informatique tourne sur un ordinateur, des étapes d'un procédé de commande et/ou de régulation d'un appareil de séparation de substances sont exécutées, l'appareil de séparation de substances comprenant plusieurs colonnes de chromatographie (2, 3, 4, 5) reliées les unes aux autres et étant prévu pour la séparation d'une première substance à partir d'un mélange de substances, le mélange de substances présentant au moins une deuxième substance, qui subit, lors de la réalisation d'une chromatographie, une rétention supérieure à celle de la première substance, et une troisième substance, qui subit, lors de la chromatographie, une rétention inférieure à celle de la première substance, l'appareil (1) étant alimenté en mélange de substances et en un éluant et la deuxième substance étant prélevée en tant qu'extrait et la première substance étant prélevée en tant que raffinat, **caractérisé en ce que** des flux de substances, en particulier les flux d'alimentation et/ou d'évacuation, du mélange de substances, de l'éluant, du raffinat et/ou de l'extrait dans l'appareil (1) sont réglés de telle sorte que la troisième substance est prélevée en tant qu'extrait.
